# EUROPÄISCHE PATENTANMELDUNG

(11) **EP 1 913 976 A1**
(43) Veröffentlichungstag der Anmeldung: **23.04.2008**
(21) Anmeldenummer: 07117428.8
(22) Anmeldetag: 27.09.2007
(51) Int. Cl.: A61Q 11/00, C07C 237/22, A61K 8/42

(54) **N-alpha-(Menthancarbonyl)aminosäureamide und deren Verwendung als physiologische Kühlwirkstoffe**

(30) Priorität: 18.10.2006 US 829953 P
(71) Anmelder: Symrise GmbH & Co. KG, 37603 Holzminden (DE)
(72) Erfinder: Ley, Jakob, 37603, Holzminden (DE)
(74) Vertreter: Eisenführ, Speiser & Partner

(57) **Zusammenfassung**

Die vorliegende Erfindung betrifft bestimmte *N*^{α}-(Menthancarbonyl)aminosäureamide der der Formel (I): 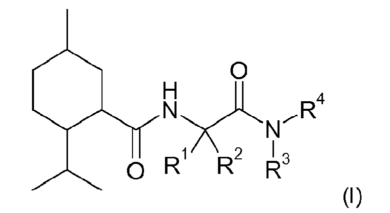 und deren Gemische sowie die Verwendung der bestimmten *N*^{α}-(Menthancarbonyl)aminosäureamide und deren Gemischen als physiologische Kühlwirkstoffe.

## Beschreibung

Die Erfindung betrifft bestimmte N^{α}-(Menthancarbonyl)aminosäureamide und deren Gemische, die eine physiologische Kühlwirkung auf der Haut und/oder einer Schleimhaut bewirken können. Sie betrifft auch Mischungen und Zubereitungen, die die N^{α}-(Menthancarbonyl)aminosäureamide in ausreichender Menge enthalten, so dass eine Kühlwirkung auf der Haut und/oder Schleimhäuten erzeugt wird. Sie betrifft außerdem die Verwendung der genannten Verbindungen als Kühlsubstanz bzw. zur Herstellung eines Arzneimittels sowie ein Verfahren zum Erreichen einer physiologischen Kühlwirkung auf der Haut und/oder Schleimhäuten.

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Gemische verwendet werden.

Der bekannteste Kühlwirkstoff ist L-Menthol, der aber einige Nachteile besitzt, z. B. starker Geruchseindruck, eine hohe Flüchtigkeit und in höheren Konzentrationen einen bitteren und/oder scharfen Eigengeschmack. Der Einsatz von L-Menthol kann somit in bestimmten Aromakompositionen, insbesondere solchen, die nicht in Richtung (Pfeffer)Minz-Aroma gehen, unerwünscht sein.

Es wurden schon früher Untersuchungen durchgeführt, die auf starke Kühlwirkstoffe ohne Aromaeffekt hinzielen. So wurden z.B. Milchsäureester von Menthol(en) gemäß DE 2 608 226 und gemischte Carbonate mit Menthol(en) und Polyolen gemäß DE 4 226 043 beschrieben, die zwar eine starke Kühlwirkung haben, deren Hydrolyse in wässrigen Medien aber zu dem stark riechenden Menthol führen können. Menthonketale gemäß EP 0 507 190 B1 sind starke Kühlwirkstoffe, die jedoch in sauren Medien Menthon abspalten können und aufgrund dessen Aromawert (starker Eigengeschmack und niedriger Schwellenwert) nicht breit eingesetzt werden können.

Menthylmonoester von Disäuren nach US 5,725,865 und US 5,843,466 sind zwar interessante natürlich vorkommende Alternativen, können aber in sensorischen Test nicht die Stärke der vorher beschriebenen Kühlwirkstoffe erreichen. Zudem sind sie als Ester auch empfindlich gegen Hydrolyse.

Mentholpolyolether z.B. aus EP 1,398,306 sind zwar stabiler gegen Hydrolyse, sind aber vom Kühleindruck her eher schwächer.

Von H.R. Watson, R. Hems, D.G. Rowsell und D.J. Spring, J. Soc. Cosmet. Chem. 1978, 29, 185-200 wurden die Ergebnisse einer Studie an ca. 1200 Verbindungen präsentiert, bei der die Verbindungen L-Menthancarbonsäure-N-ethylamid ("WS3") und insbesondere *N*^{α}-(L-Menthancarbonyl)-glycinethylester ("WS5") als die stärksten Kühlwirkstoffe gefunden wurden. Letzterer hat bei starker Wirkung aber den Nachteil, hydrolyseempfindlich zu sein und dabei die entsprechende freie Säure *N*^{α}-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlwirkstoffen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten (z.B. die Menthancarbonsäure-N-(alkyloxyalkyl)amide nach JP 2004059474) oder sind zusätzlich stark reizend (WS5: N-[[5-Methyl-2-(1-methylethyl)cyclohexyl]carbonyl]glycinethylester, US 2005/0222256).

*N*^{α}-(Menthancarbonyl)alkyloxyalkylamide wurden in JP 2004059474 beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar.

Es war daher die primäre Aufgabe der vorliegenden Erfindung, neue Verbindungen bzw. Gemische von Verbindungen anzugeben, die eine starke physiologische Kühlwirkung besitzen, eine gute und im Vergleich zu bekannten Kühlwirkstoffen verbesserte (Hydrolyse-)Stabilität aufweisen und dabei in Nahrungs- und/oder Genussmitteln und/oder Mundpflegeprodukten und/oder (oralen) pharmazeutischen Zubereitungen und/oder kosmetischen Zubereitungen als Kühlsubstanzen (Kühlwirkstoffe) eingesetzt werden können. Die anzugebenden Verbindungen bzw. Verbindungsgemische sollten vorzugsweise einen möglichst schwachen Eigengeschmack zeigen, insbesondere wenig oder gar nicht bitter schmecken sowie möglichst nicht reizend sein.

Diese primäre Aufgabe wird erfindungsgemäß gelöst durch eine Verbindung (*N*^{α}-(Menthancarbonyl)aminosäureamid) oder ein Gemisch von Verbindungen der allgemeinen Formel (I): 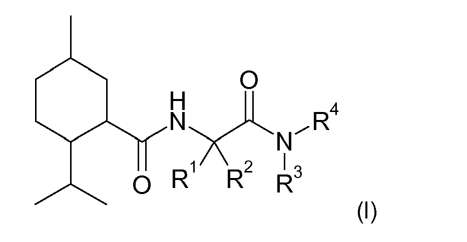 wobei
- R¹ und R²: jeweils unabhängig voneinander Wasserstoff oder einen
- (a): linearen, verzweigten oder cyclischen,
- (b): gesättigten oder ungesättigten,
- (c): unsubstituierten, ein- oder mehrfach substituierten Kohlenwasserstoffrest mit
- (d): 1 bis 5 Kohlenstoffatomen bedeuten, wobei
- (e): die Kohlenwasserstoffreste R¹ und R² auch über eine Einfachbindung oder über eine Gruppe -O-, -S- oder -NH- verknüpft sein können und dabei bevorzugt einen 3- bis 7-gliedrigen Ring ausbilden,
und
- R³ und R⁴: jeweils unabhängig voneinander Wasserstoff oder einen
- (a): linearen, verzweigten oder cyclischen,
- (b): gesättigten oder ungesättigten Kohlenwasserstoffrest mit
- (c): 1 bis 10 Kohlenstoffatomen bedeuten, wobei
- (d): die Kohlenwasserstoffreste R³ und R⁴ auch über eine Einfachbindung oder über eine Gruppe -O-, -S- oder -NH- verknüpft sein können und dabei bevorzugt einen 3 bis 7-gliedrigen heterocyclischen Ring ausbilden,
oder
- R³ und R⁴: unabhängig voneinander jeweils Wasserstoff oder einen
- (a): linearen oder verzweigten,
- (b): durch eine Gruppe R⁵-X- substituierten Kohlenwasserstoffrest mit
- (c): 1 bis 10 Kohlenstoffatomen bedeuten, wobei jedes X falls vorhanden jeweils unabhängig von dem anderen X falls vorhanden
- (d): Sauerstoff, Schwefel oder eine Gruppe -NR⁶- darstellt,
und
wobei jeder R⁵ und jeder R⁶ falls vorhanden jeweils unabhängig voneinander Wasserstoff oder einen
(a) linearen, verzweigten oder cyclischen,
(b) gesättigten oder ungesättigten Kohlenwasserstoffrest mit
(c) 1 bis 5 Kohlenstoffatomen bedeuten,
und
wobei bevorzugt die jeweilige Verbindung der Formel (I) einzeln oder eine der Verbindungen der Formel (I) im Gemisch von Verbindungen der Formel (I) unabhängig von den anderen Verbindungen der Formel (I) als einzelnes Stereoisomer (Enantiomere und Diastereomere) oder als bestimmtes Gemisch verschiedener Stereoisomere vorliegt.

Bestimmte Gemische sind im Zusammenhang mit dieser Anmeldung insbesondere Gemische, bei denen ein einzelnes der Formel (I) entsprechendes Stereoisomer 90 bis 100 mol.-% bezogen auf die Gesamtzahl der zu diesem Stereoisomer isomeren Verbindungen darstellt.

In diesem Text ist ein linearer, verzweigter oder cyclischer, gesättigter oder ungesättigter Kohlenwasserstoffrest mit 1 bis 5 Kohlenstoffatomen, der seinerseits ein- oder mehrfach substituiert sein kann, insbesondere Methyl-, Ethyl-, Propyl-, 2-Propyl-, 2-Methylprop-1-yl, 2-Methylpropyl-, 2-Butyl-, 2-Methylbutyl- Rest oder ein Methylrest, der seinerseits durch Arylreste, Heterocyclylreste, H₂N-, Guanidino-, (Iso-)Harnstoff-, HO-, Alkoxy-, HS-, Alkylthio-, (subst.) Sulfid-, (subst.) Disulfid-, (subst.) Phosphat-, -COOH, -CONH₂, substituiert sein kann, wobei die Substituenten bevorzugt zusammem mit dem Metyhlrest 4-Aminobutyl-, 3-Guanidinopropyl, 3-Aminopropyl-, 3-Ureidopropyl-, Indol-3-ylmethyl-, 2-Carboxyethyl-, Carboxymethyl-, 2-(Aminocarbonyl)ethyl-, (Aminocarbonyl)methyl, Thiomethyl-, 2-Thioethyl-, 2-Methylthioethyl, Hydroxymethyl, 1-Hydroxyethyl, Phenylmethyl- und 4-Hydroxyphenylmethylergeben.

Ein linearer, verzweigter oder cyclischer, gesättigter oder ungesättigter Kohlenwasserstoffrest mit 1 bis 5 Kohlenstoffatomen ist in diesem Text bevorzugt insbesondere Methyl-, Ethyl-, 2-Propyl, Cyclopropyl, Propyl, Butyl, Cyclobutyl, 2-Butyl, 2-Methylprop-1-yl, 2-Methylprop-2-yl, Pentyl, Cyclopentyl, 2-Pentyl und 3-Pentyl.

Ein linearer, verzweigter oder cyclischer Kohlenwasserstoffrest mit 1 bis 10 Kohlenstoffatomen ist in diesem Text insbesondere Methyl-, Ethyl-, 2-Propyl, Cyclopropyl, Propyl, Butyl, Cyclobutyl, 2-Butyl, 2-Methylprop-1-yl, 2-Methylprop-2-yl, Pentyl, Cyclopentyl, 2-Pentyl, 3-Pentyl, Hexyl, Cyclohexyl, 2-Hexyl und 3-Hexyl, Heptyl, Octyl, Isooctyl, Nonyl, Decyl.

Ein linearer oder verzweigter, durch eine Gruppe R⁵-X- substituierter Kohlenwasserstoffrest mit 1 bis 10 Kohlenstoffatomen ist in diesem Text insbesondere ein Kohlenwasserstoffrest mit 1 bis 5 Kohlenstoffatomen, z.B. Methylen, Ethylen, 1,2-Propylen, 1,3-Propylen, 1,2-Butylen, 1,3-Butylen, 1,4-Butylen, 1,2-Pentylen, 1,3-Pentylen, 1,4-Pentylen und 1,5-Pentylen.

Bevorzugt ist eine erfindungsgemäße Verbindung der Formel (I) oder ein erfindungsgemäßes Gemisch von Verbindungen der Formel (I): 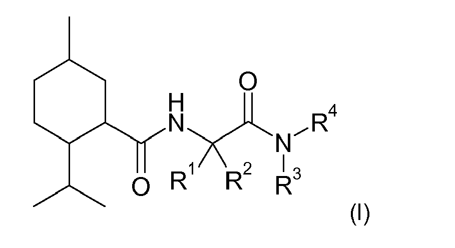 wobei R¹ Wasserstoff bedeutet
und/oder
- R²: Wasserstoff oder einen
- (a): linearen, verzweigten oder cyclischen,
- (b): gesättigten oder ungesättigten,
- (c): unsubstituierten, ein- oder mehrfach substituierten Kohlenwasserstoffrest mit
- (d): 1 bis 5 Kohlenstoffatomen bedeutet
und/oder
- R³ und R⁴: jeweils unabhängig voneinander Wasserstoff oder einen
- (a): linearen, verzweigten oder cyclischen Kohlenwasserstoffrest mit
- (b): 1 bis 10 Kohlenstoffatomen bedeuten, wobei
- (c): bevorzugt die Kohlenwasserstoffreste R³ und R⁴ über eine Einfachbindung oder über eine Gruppe -O-, oder -NH- verknüpft sind und dabei einen Azirin-, Azetidin-, Pyrrolidin-, Imidazolidin, Piperidin-, Pyrazolidin-, Diazan- oder Morpholin-Ring ausbilden
oder
- R³ und R⁴: unabhängig voneinander jeweils Wasserstoff oder einen
- (a): linearen oder verzweigten,
- (b): durch eine Gruppe R⁵-X- substituierten Kohlenwasserstoffrest mit
- (c): 1 bis 10 Kohlenstoffatomen bedeuten, wobei bevorzugt jedes X falls vorhanden jeweils unabhängig von dem anderen X falls vorhanden
- (d): Sauerstoff, Schwefel oder eine Gruppe -NR⁶- darstellt und wobei bevorzugt jeder R⁶ falls vorhanden Wasserstoff bedeutet.

Weiter bevorzugt ist eine erfindungsgemäße Verbindung oder ein erfindungsgemäßes Gemisch
wobei die Verbindung der Formel (I) (*N*^{α}-(Menthancarbonyl)aminosäureamid) oder eine Verbindung der Formel (I) im Gemisch von Verbindungen der Formel (I) unabhängig von den anderen Verbindungen der Formel (I) hinsichtlich ihrer Stereochemie entsprechend der Formel (la) 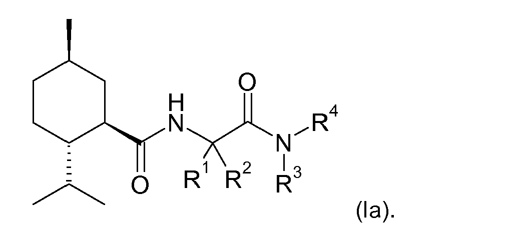 zu 90 -100 Mol-% festgelegt ist, wobei
in der Formel (la) die Reste R¹, R², R³ und R⁴ jeweils die Bedeutung der entsprechenden Reste der Formel (I) haben
und wobei bevorzugt alle Verbindungen der Formel (I) in dem Gemisch hinsichtlich ihrer Stereochemie zu 90 -100 Mol-% entsprechend der Formel (la) festgelegt sind.

Besonders bevorzugt ist eine erfindungsgemäße Verbindung (N^{α}-(Menthancarbonyl)aminosäureamid) der Formel (I) oder ein erfindungsgemäßes Gemisch von Verbindungen der Formel (I), wobei in der Formel (I) 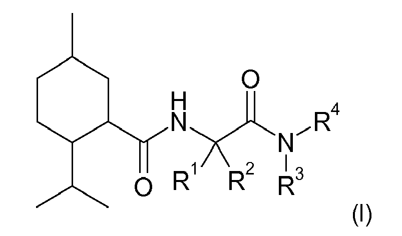
- R¹: Wasserstoff bedeutet und/oder
- R²: Wasserstoff oder einen Methyl-, Ethyl-, Propyl-, 2-Propyl-, 2-Methylpropyl-, 2- Butyl-, 2-Methylbutyl-, 4-Aminobutyl-, 3-Guanidinopropyl, 3-Aminopropyl-, 3-Ureidopropyl-, Indol-3-ylmethyl-, 2-Carboxyethyl-, Carboxymethyl-, 2-(Aminocarbonyl)ethyl-, (Aminocarbonyl)methyl-, Thiomethyl-, 2-Thioethyl-, 2-Methylthioethyl-, Hydroxymethyl-, 1-Hydroxyethyl-, Phenylmethyl- oder 4-Hydroxyphenylmethyl-Rest bedeutet
und/oder
- R³: Wasserstoff oder einen Methyl- oder Ethyl- Rest bedeutet,
und/oder
- R⁴: Wasserstoff oder einen Methyl-, Ethyl-, 2-Propyl-, Cyclopropyl-, Propyl-, Butyl-, Cyclobutyl-, 2-Butyl-, 2-Methylprop-1-yl, 2-Methylprop-2-yl-, Pentyl-, Cyclopentyl-, 2- Pentyl-, 3-Pentyl-, Hexyl-, Cyclohexyl-, 2-Hexyl- und 3-Hexyl-, Heptyl-, Octyl-, Isooctyl-, Nonyl- oder Decyl-Rest bedeutet,
oder
- R⁴: einen durch eine Gruppe R⁵-X- substituierte n Methylen -, Ethylen-, 1,2-Propylen-, 1,3-Propylen-, 1,2-Butylen-, 1,3-Butylen-, 1,4-Butylen-, 1,2-Pentylen-, 1,3-Pentylen-, 1,4-Pentylen- oder 1,5-Pentylen-Rest bedeutet, wobei X Sauerstoff darstellt und R⁵ Wasserstoff oder einen Methyl- oder Ethyl-Rest bedeutet,
und/oder
wobei bevorzugt die Verbindung oder die Verbindungen der Formel (I) zu 90 - 100 Mol-% hinsichtlich ihrer Stereochemie entsprechend der Formel (la): 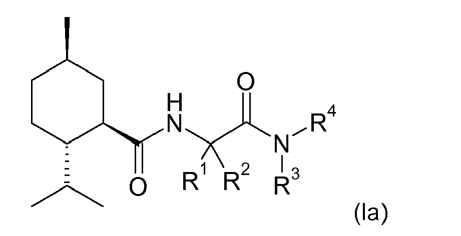 festgelegt sind und wobei die jeweiligen Reste R die vorgenannte Bedeutung besitzen und wobei bevorzugt die Verbindung oder die Verbindungen der Formel (I) zu 90 - 100 Mol-% hinsichtlich ihrer Stereochemie entsprechend der Formel (la) festgelegt sind.

Ganz besonders bevorzugt ist eine erfindungsgemäße Verbindung oder ein erfindungsgemäßes Gemisch von Verbindungen der Formel (I) (*N*^{α}-(Menthancarbonyl)aminosäureamide) 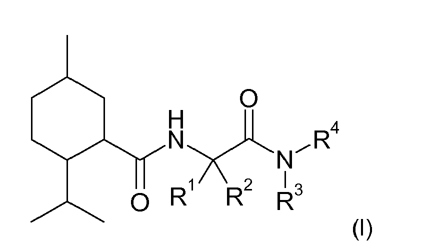 wobei
- R¹: Wasserstoff bedeutet
und/oder
- R²: Wasserstoff oder einen Methyl-, Ethyl-, Propyl- oder 2-Propyl -Rest bedeutet
und/oder
- R³: ein Wasserstoff oder einen Methyl- oder Ethyl- Rest bedeutet,
und/oder
- R⁴: Wasserstoff oder einen Methyl-, Ethyl-, 2-Propyl-, Cyclopropyl-, Propyl-, Butyl-, Cyclobutyl-, 2-Butyl-, 2-Methylprop-1-yl, 2-Methylprop-2-yl-, Pentyl-, Cyclopentyl-, 2-Pentyl-, 3-Pentyl-, Hexyl-, Cyclohexyl-, 2-Hexyl- und 3-Hexyl-, Heptyl-, Octyl-, Isooctyl-, Nonyl- oder Decyl-Rest bedeutet,
oder
- R⁴: einen durch eine Gruppe R⁵-X- substituierten Methylen-, Ethylen-, 1,2-Propylen- oder 1,3-Propylen- bedeutet,
wobei X Sauerstoff darstellt und
- R⁵: Wasserstoff oder einen Methylrest bedeutet,
und wobei bevorzugt die erfindungsgemäßen N^{α}-(Menthancarbonyl)aminosäureamide der Formel (I) zu 90 bis 100 mol.-% hinsichtlich ihrer Stereochemie entsprechend der Formel (la) 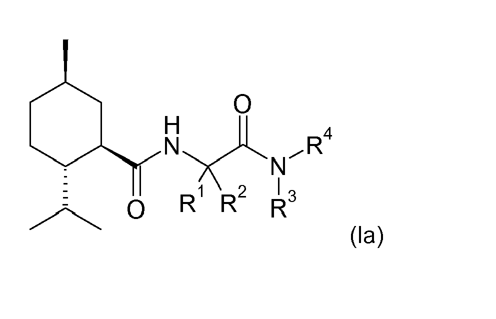 festgelegt sind, wobei die jeweiligen Reste R in der Formel (la) die vorgenannte Bedeutung besitzen.

Als besonders bevorzugte Einzelverbindungen seien genannt:
L-*N*^{α}-(Menthancarboxyl)glycin-*N*-isobutylamid (Verbindung 1)
L-*N*^{α}-(Menthancarboxyl)glycin-*N,N*-dimethylamid (Verbindung 2)
L-*N*^{α}-(Menthancarboxyl)glycin-*N*-ethylamid (Verbindung 3)
L-*N*^{α}-(Menthancarboxyl)glycin-*N*-ethanolamid (Verbindung 4)
L-*N*^{α}-(Menthancarboxyl)-L-alanin-*N*-ethylamid (Verbindung 5)

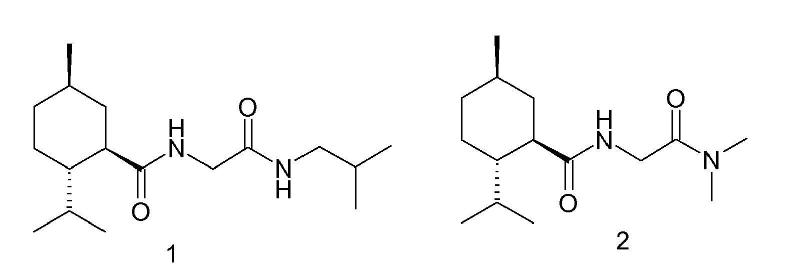

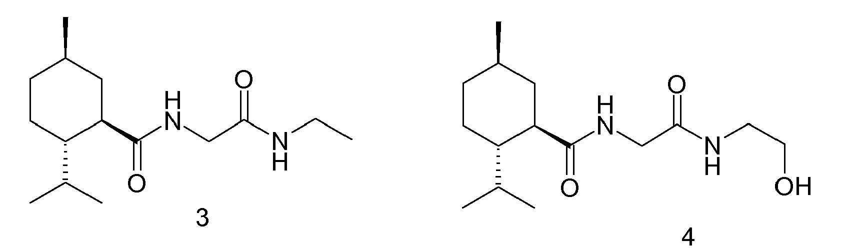

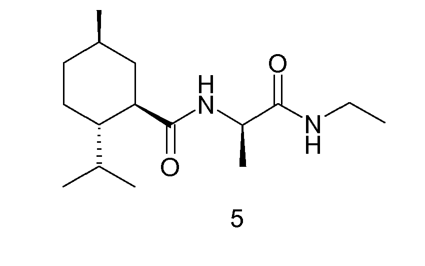

Die Erfindung basiert auf der überraschenden Erkenntnis, dass die erfindungsgemäßen Verbindungen, (*N*^{α}-(Menthancarbonyl)aminosäureamid) der Formeln (I) und (Ia)) sowie deren Gemische ein starkes und langanhaltendes Gefühl der Kälte auf der Haut oder Schleimhaut verursachen, insbesondere auf den Schleimhäuten des Mund-, Nasen- und Rachenraumes. Dabei zeigen die besagten Verbindungen keine anderen trigeminalen Effekte wie Schärfe, Tingling oder Betäubung und sind nicht bitter. Gleichzeitig sind die erfindungsgemäßen Verbindungen im Rahmen der üblichen Formulierungen und Zubereitungsbedingungen im Bereich von pH 1 bis pH 12, insbesondere im Bereich von pH 4 bis pH 9, bezogen auf wasserhaltige Zubereitungen, hydrolysestabil, so dass die erfindungsgemäßen Verbindungen und Gemische in Zubereitungen lange haltbar sind, so dass auch die jeweilige Zubereitung selbst lange haltbar ist.

Hydrolysestabil bedeutet im Zusammenhang mit diesem Text, dass die untersuchten Verbindungen bei 40°C, bei pH9 und/oder pH3, bei Tageslicht und bei einem Wassergehalt von mindestens 5 Gew.% nach 6 Monaten zu weniger als 10 Mol-% (bevorzugt nicht signifikant) hydrolisiert werden und darüber hinaus bevorzugt unter den üblichen Herstellungsbedingungen für die in diesem Text genannten Zubereitungen zu weniger als 10%, (nochmals bevorzugt nicht signifikant) Hydrolysereaktionen zeigen.

Die Erfindung betrifft auch eine Mischung bestehend aus/umfassend
(a) einer/eine erfindungsgemäße(n) Verbindung oder einem/ein erfindungsgemäßen/erfindungsgemäßes Gemisch von Verbindungen
   sowie
(b) einem/einen oder mehreren/mehrere weitere(n) Stoffe(n) mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
   und/oder
(c) einem/einen oder mehreren/mehrere Aromastoffe ohne physiologische Kühlwirkung
   und/oder
(d) einem/einen oder mehreren/mehrere trigeminal oder mundwässernd wirksame(n) Stoffe(n) ohne physiologische Kühlwirkung.

Besonders bevorzugt ist eine erfindungsgemäße Mischung, umfassend als Bestandteil (b) einen oder mehrere weitere Stoffe mit physiologischer Kühlwirkung, wobei diese keinen geschmacklichen Effekt und keine Aromawirkung verursachen, sondern lediglich eine Kühlwirkung (im Wesentlichen) ohne weiteren sensorischen Effekt. Hierdurch wird vermieden, dass das Aromaprofil der erfindungsgemäßen Mischung beispielsweise in Richtung "Minze" (Pfefferminze) gebunden wird.

Ganz besonders bevorzugt ist eine erfindungsgemäße Mischung umfassend als Bestandteil (c) einen oder mehrere Aromastoffe ohne physiologische Kühlwirkung und/oder als Bestandteil (d) eine oder mehrere Verbindungen, der oder die unabhängig voneinander oder gemeinsam zusätzlich einen geschmacksmodulierenden Effekt und/oder einen trigiminalen und/oder einen mundwässernden Reiz verursachen, wobei der trigiminale Reiz bevorzugt keine physiologische Kühlwirkung darstellt. Insbesondere besitzen solche erfindungsgemäße Mischungen, die die letztgenannten Bestandteile (c) und (d) gleichzeitig enthalten eine angenehme Kühlwirkung und ein ausgeglichenes sensorisches Profil bei gleichzeitig hohem Impact, d.h. hohem geschmacklichen Ersteindruck.

Der eine bzw. die mehreren weiteren Stoffe mit physiologischer Kühlwirkung, die als Bestandteil (b) in einer erfindungsgemäßen Mischung eingesetzt werden können, werden dabei vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), andere als in der vorliegenden Erfindung genannte Menthancarbonsäureamide (z.B. Menthancarbonsäure-N-ethylamid [WS3], *N*^{α}-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Der oder die mehreren weiteren Stoffe mit physiologischer Kühlwirkung, die als Bestandteil (b) einer erfindungsgemäßen Mischung eingesetzt werden können, sind insbesondere vorzugsweise Stoffe, welche zumindest im Wesentlichen eine physiologische Kühlwirkung verursachen, ohne gleichzeitig eine geschmackliche Wirkung zu verursachen. Solche bevorzugten Stoffe sind: Menthylether (z.B. (I-Menthoxy)-1,2-propandiol, (I-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäße Menthancarbonsäureamide (z.B. Menthancarbonsäure-N-ethylamid [WS3], N^{α}-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Bestimmte Mischungen von Menthancarbonsäureamiden mit Kühlwirkstoffen wie acyclischen Carbonsäureamiden und L-Menthyllactat sowie gegebenenfalls weiteren Kühlwirkstofffen oder trigeminalen Reizstoffen sind zum Beispiel in US 2005/0117811 beschrieben; es findet sich dort jedoch kein Hinweis auf den Einsatz der erfindungsgemäßen *N*^{α}-(Menthancarbonyl)aminosäureamide (Verbindungen der Formeln (I) und (Ia)). Auch die in der US 2005/0117811 genannten Substituenten geben keinen Hinweis in Richtung auf die erfindungsgemäße Verwendung.

Bevorzugte Aromastoffe ohne physiologische Kühlwirkung sind die Aromastoffe, die zu ihrem eigentlichen geruchlichen Aromawert auch einen Geschmackseindruck, einen geschmacksmodulierenden Effekt oder einen trigeminalen, aber nicht-kühlenden oder auch einen mundwässernden Reiz verursachen. Bevorzugte Geschmackseindrücke sind süß, Umami, bitter, salzig und sauer; bevorzugte geschmacksmodulierende Effekte sind bittermaskierende, umami-verstärkende, süß-verstärkende, salz-verstärkende und sauer-maskierende Effekte; bevorzugte trigeminale Reize sind Schärfe, Wärme, Kribbeln und Stechen. Besonders bevorzugte Aromastoffe ohne physiologische Kühlwirkung sind z.B. Pellitorine nach WO 04 000,787 oder US 2004/0241312 und Alkamide nach DE 103 51,422.

Die Synthese der erfindungsgemäßen Verbindungen der Formel (1) bzw. die Synthese entsprechender Gemische wird vorzugsweise durch Umsetzung der entsprechenden *N*^{α}-(Menthancarbonyl)aminosäure, eines entsprechenden *N*^{α}-(Menthancarbonyl)aminosäurechlorids oder eines anderen entsprechenden aktivierten *N*^{α}-(Menthancarbonyl)aminosäurederivats mit einem entsprechenden Amin oder einem entsprechenden Salz erreicht.

Vorzugsweise wird hierbei eine mehr als 90 Gew.-%, vorzugsweise mehr als 95 Gew.-%, (L)-*N*^{α}-(Menthancarbonyl)aminosäure umfassende *N*^{α}-(Menthancarbonyl)aminosäure oder ein daraus hergestelltes (L)-*N*^{α}-(Menthancarbonyl)aminosäurechlorid oder ein daraus hergestelltes anderes aktiviertes (L)-*N*^{α}-(Menthancarbonyl)aminosäurederivat eingesetzt. Die Synthese kann gemäß an sich bekannter Acylierungsmethoden erfolgen.

Die Erfindung betrifft auch ein entsprechendes Verfahren zur Herstellung einer erfindungsgemäßen Verbindung oder zur Herstellung eines erfindungsgemäßen Gemisches von Verbindungen.

Ein bevorzugter Syntheseweg kann anhand des nachfolgenden Reaktionsschemas verdeutlicht werden, welches zu einer Verbindung der Formel (I) führt. Ein entsprechender Syntheseweg ist bevorzugt für die Herstellung einer Verbindung der Formel (la).

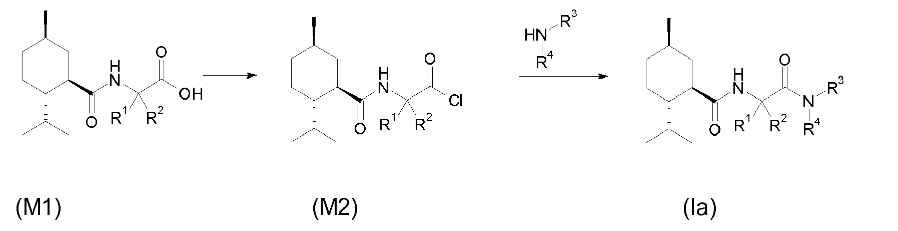

Dabei kann die (L)-*N*^{α}-(Menthancarbonyl)aminosäure (M1) beispielsweise nach bekannten Verfahren mittels SOCl₂, (COCl)₂ oder PCl₃ in das entsprechende Säurechlorid (M2) überführt werden.
Optional kann diese Umsetzung in Gegenwart von anderen (Hilfs)Stoffen oder Additiven erfolgen, z.B. in Gegenwart von
(i) einem oder mehreren Lösungsmitteln bzw. Verdünnungsmitteln (z.B. Wasser, Wasser/1,4-Dioxan-Gemische, Tetrahydrofuran, Wasser-Tetrahydrofuran-Gemische, andere Ether, Chloroform, Methylenchlorid, Ethylacetat, Aceton, Aceton-Wasser-Gemische, Alkanen, Alkoholen)
   und/oder
(ii) anorganischen oder organischen Hilfsbasen (z.B. Triethylamin, andere trialkylierte Amine, Carbonate, Hydroxide, basische Oxide oder Hydrogencarbonate von Alkali- und/oder Erdalkalimetallen, basische lonentauscher),
   und/oder
(iii) Phasentransferkatalysatoren (z.B. peralkylierte/perarylierte Ammoniumsalze oder Phosphoniumsalze, Kronenether),
   und/oder
(iv) Enzymen und/oder geeigneten Mikroorganismen (insbesondere hydrolytische Enzyme wie Lipasen, Amidasen, Peptidasen).

Die Rohprodukte der Synthese werden vorzugsweise durch physikalische, gegebenenfalls auch enantioselektive oder enantiospezifische Trennmethoden, z.B. Extraktion, Verteilungsmethoden, Kristallisation, Destillation, Chromatographie, Sublimation, Wasserdampfdestillation, Umkehrosmose, Permeation oder dergleichen aufgereinigt bzw. angereichert, wobei die Trennmethode vorzugsweise so gewählt ist, dass nach der Trennoperation die Stereochemie der erfindungsgemäßen *N*^{α}-(Menthancarbonyl)aminosäure der Formel (la) (soweit die Formel (Ia) die Stereochemie festlegt) zu einem Anteil von 90-100 Mol-%, bevorzugt 95 - 100 Mol-%, entspricht, bezogen auf die Gesamtmenge der in dem aufgereinigten Produkt vorliegenden Verbindungen der Formel (I).

Die Erfindung betrifft auch der Ernährung, der Mundhygiene oder dem Genuss dienende oder pharmazeutische oder kosmetische Zubereitungen, die eine zum Erreichen einer physiologischen Kühlwirkung auf der Haut und/oder Schleimhaut ausreichende Menge (a) einer erfindungsgemäßen Verbindung oder eines erfindungsgemäßen Gemisches (vorzugsweise in einer als bevorzugt angegebenen Ausgestaltung) oder (b) einer erfindungsgemäßen Mischung (vorzugsweise in einer als bevorzugt angegebenen Ausgestaltung) umfassen. Insbesondere soll die eingesetzte Menge der Verbindung, des Gemisches bzw. der Mischung zum Erreichen einer physiologischen Kühlwirkung auf der Schleimhaut im Mund-, Nasen- und/oder Rachenraum ausreichen.

Bevorzugte erfindungsgemäße Zubereitungen umfassen übliche Grund-, Hilfs-und Zusatzstoffe für der Ernährung, der Mundhygiene oder dem Genuss dienende oder pharmazeutische oder kosmetische Zubereitungen. Bevorzugte erfindungsgemäße Zubereitungen enthalten 0,0001 Gew.-% bis 20 Gew.-%, bevorzugt 0,0001 bis 10 Gew.-%, besonders bevorzugt 0,001 Gew.-% bis 0,5 Gew.-% an Verbindungen der Formel (I), bezogen auf das Gesamtgewicht der Zubereitung. Weitere Bestandteile, insbesondere Verbindungen der Formel (la) sowie Bestandteile (b), (c) und/oder (d) (wie oben beschrieben) sowie weitere übliche Grund-, Hilfs- und Zusatzstoffe können in Mengen von 0,0000001 bis 99,99 Gew.-%, vorzugsweise 10 bis 80 Gew.-%, bezogen auf das Gesamtgewicht der Zubereitung, enthalten sein. Ferner können die erfindungsgemäßen Zubereitungen Wasser in einer Menge bis zu 99,99 Gew.-%, vorzugsweise 5 bis 80 Gew.-%, bezogen auf das Gesamtgewicht der Zubereitung, enthalten.

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kaffee, Tee, Wein, weinhaltige Getränke, Bier, bierhaltige Getränke, Liköre, Schnäpse, Liköre, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett-und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nicht überzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundhygiene dienende Zubereitungen sind insbesondere Zahnpflegemittel wie Zahnpasten, Zahngele, Zahnpulver, Mundwässer, Kaugummis und andere Mundpflegemittel.

Zahnpflegemittel (als Basis für der Mundpflege dienende Zubereitungen), welche die erfindungsgemäße Verbindungen, Gemische oder Mischungen enthalten, umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Natrium-Cyclamat, Sucralose, Acesulfam-K oder Zuckeralkohole, Geschmackskorrigenzien für unangenehme Geschmackseindrücke wie z.B Hydroxyflavanone nach US 2002/0188019, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), die erfindungsgemäße Verbindungen, Gemische oder Mischungen enthalten, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugte erfindungsgemäße pharmazeutische Zubereitungen im Sinne der Erfindung sind orale Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Erfindungsgemäße kosmetische Zubereitungen können z. B. in einer der folgenden Formen vorliegen: Seife, Syndet, flüssiges Wasch-, Dusch-, oder Badepräparat, Emulsion (als Lösung, Dispersion, Suspension, Creme, Lotion oder Milch je nach Herstellungsverfahren und Inhaltsstoffen vom Typ "Wasser-in-Öl" (W/O), "Öl-in-Wasser" (O/W) oder multiple Emulsion, PIT Emulsion, Emulsionsschaum, Mikroemulsion, Nanoemulsion, Pickering Emulsion), Salbe, Paste, Gel (inklusive Hydro-, Hydrodispersions-, Oleogel), Öl, Toner, Balsam, Serum, Puder, Eau de Toilette, Toilette, Eau de Cologne, Perfum, Wachs, als Stift, Roll-On, (Pump-)Spray, Aerosol (schäumend, nicht schäumend oder nachschäumend), Fusspflegemittel (inklusive Keratolytika, Desodorant), Bartreinigungs- oder -pflegemittel, Insekten abwehrendes Mittel, Sonnenschutzmittel, Aftersun-Präparat, Rasiermittel (z.B. Rasierschäume, - seifen oder -gele) oder After-Shave (Balm, Lotion), Haarentfernungsmittel, Haarpflegemittel wie z.B. Shampoo (z.B. 2-in-1 Shampoo, Anti-Schuppen-Shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (z.B. Gel oder Wachs), Blondiermittel, Haaraufheller, Haarconditioner, Haarmousse, Haartönung, Haarfärbemittel (z.B. temporäre, direktziehende, semipermanente, permanente Haarfärbemittel), Nagelpflegemittel wie z.B. Nagellack und Nagellackentferner, Deodorant und / oder Antitranspirant, Mundwasser, Munddusche, Make-Up, Make-Up-Entferner, Augenpflege, Lippenkosmetika, Lippenpflegemittel, dekorative Kosmetik (z.B. Puder, Lidschatten, Kajalstift, Lippenstift), Badeartikel (z.B. Kapsel), oder Maske.

Erfindungsgemäße Zubereitungen, die erfindungsgemäße Verbindungen, ein erfindungsgemäßes Gemisch oder eine erfindungsgemäße Mischung umfassen, werden vorzugsweise hergestellt, indem die Verbindung, das Gemisch oder die Mischung, z. B. eine Mischung, die neben einer erfindungsgemäßen Verbindung einen festen oder flüssigen Trägerstoff umfasst, in eine Basis-Zubereitung eingearbeitet wird. Vorteilhafterweise werden zunächst als Lösung vorliegende erfindungsgemäße Mischungen, die eine erfindungsgemäße Verbindung umfassen, durch Sprühtrocknung in eine feste Zubereitung überführt.

Erfindungsgemäße Zubereitungen können gemäß einer alternativen bevorzugten Ausführungsform hergestellt werden, indem die erfindungsgemäßen Verbindungen, Gemische bzw. Mischungen, gegebenenfalls mit weiteren Bestandteilen der erfindungsgemäßen Zubereitung, zunächst in Emulsionen, in Liposomen, z. B. ausgehend von Phosphatidylcholin, in Microsphären, in Nanosphären oder auch in Kapseln, Granulaten oder Extrudaten aus einer für Lebens- und Genussmittel oder kosmetische Zubereitungen geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatine oder sonstigen Naturprodukten (z.B. Schellack) oder nichtnatürlichen Matrixmaterialien (wie Polyharnstoff) eingearbeitet werden. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und ggfs. einer geeigneter Kombination der vorgenannten Verfahren behandelt werden.

In einem weiteren bevorzugten Herstellungsverfahren werden die erfindungsgemäßen Verbindungen, Gemische bzw. Mischungen zunächst mit einem oder mehreren geeigneten Komplexbildnern, beispielsweise mit Cyclodextrinen oder Cyclodextrinderivaten, bevorzugt alpha-, beta- oder gamma-Cyclodextrin, komplexiert und in dieser komplexierten Form eingesetzt.

Besonders bevorzugt ist eine erfindungsgemäße Zubereitung, bei der die Matrix so gewählt ist, dass die erfindungsgemäßen Verbindungen, Gemische oder Mischungen, insbesondere Mischungen, die weitere Kühlwirkstoffe und/oder Aromen umfassen, verzögert von der Matrix freigegeben werden, so dass eine langanhaltende Kühlwirkung erreicht wird.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Ein weiterer Aspekt der vorliegenden Erfindung betrifft die Verwendung
(a) einer erfindungsgemäßen Verbindung oder eines erfindungsgemäßen Gemisches (wie oben definiert, vorzugsweise in einer oben beschriebenen bevorzugten Ausgestaltung),
(b) einer erfindungsgemäßen Mischung (wie oben beschrieben, vorzugsweise in einer als bevorzugt beschriebenen Ausgestaltung) oder
(c) einer erfindungsgemäßen Zubereitung (wie oben beschrieben, vorzugsweise in einer als bevorzugt angegebenen Ausgestaltung),
zum Erzeugen einer Kühlwirkung auf der Haut oder einer Schleimhaut zu
(i) anderen als therapeutischen Zwecken oder
(ii) zum Herstellen eines Arzneimittels.

Bevorzugt werden die erfindungsgemäßen Verbindungen, Gemische und/oder Mischungen zum Herstellen eines Arzneimittels, das der Bekämpfung oder der Linderung von Husten-Schnupfen-, Mund-, Nasen-, Hals- oder Rachenentzündungs-, Halsschmerz- oder Heiserkeitssymptomen dient, verwendet.

Ein weiterer Aspekt der vorliegenden Erfindung betrifft ein Verfahren zum Erreichen einer physiologischen Kühlwirkung auf der Haut und/oder einer Schleimhaut. Ein solches erfindungsgemäßes Verfahren kann zu therapeutischen oder nicht-therapeutischen (z. B. kosmetischen) Zwecken durchgeführt werden und umfasst den folgenden Schritt:
- Applizieren einer zum Erreichen einer physiologischen Kühlwirkung ausreichenden Menge
   (i) einer erfindungsgemäßen Verbindung oder eines erfindungsgemäßen Gemisches (wie oben definiert, vorzugsweise in einer oben beschriebenen bevorzugten Ausgestaltung),
   (ii) einer erfindungsgemäßen Mischung (wie oben beschrieben, vorzugsweise in einer als bevorzugt beschriebenen Ausgestaltung) oder
   (iii) einer erfindungsgemäßen Zubereitung (wie oben beschrieben, vorzugsweise in einer als bevorzugt angegebenen Ausgestaltung),
   auf die Haut und/oder eine Schleimhaut.

Ein weiterer Aspekt der Erfindung betrifft die Verwendung erfindungsgemäßer Zubereitungen, enthaltend eine erfindungsgemäße Verbindung, eine erfindungsgemäßes Gemisch oder eine erfindungsgemäße Mischung, vorzugsweise eine erfindungsgemäße Mischung, welche einen oder mehrere Aromastoffe und/oder einen oder mehrere weitere Kühlwirkstoffe (Kühlwirkstoffe, bei denen es sich nicht um eine Verbindung der allgemeinen Formel (I) handelt) umfasst, als Halbfertigwaren (sogenannte Aromamischungen) zur Aromatisierung von unter Verwendung der Halbfertigwaren gefertigten Fertigwaren.

Weitere Aspekte der vorliegenden Erfindung ergeben sich aus den nachfolgenden Beispielen sowie dem beigefügten Patentansprüchen.

### Beispiele

Die Beispiele dienen nur zur Verdeutlichung der Erfindung, ohne diese damit einzuschränken. Sofern nicht anders angegeben, beziehen sich alle Angaben auf das Gewicht.

### Beispiel 1: Synthese von L-N^{α}-(Menthancarboxyl)glycin-N-isobutylamid (Verbindung 1)

L-*N*^{α}-(Menthancarboxyl)glycinethylester ("WS 5") wurde mit Isobutylamin in Toluol mit Hilfe von Chirazym L2c2 bei 56°C umgesetzt. Nach Filtration, Eindampfen und Aufreinigung per Chromatographie konnte Verbindung 1 als kristalliner, farbloser Reinstoff erhalten werden.
¹H-NMR (400 MHz, CDCl₃, TMS): δ = 6,82 (1 H, br s, NH), 6,72 (1H, br s, NH), 3.92 (2H, d, 5,3 Hz), 3.08 (2H, dd, 6.7 Hz, 6.1 Hz), 2.13 (1 H, ddd, 11.7 Hz, 11,4 Hz, 3,4 Hz), 1,82-1,62 (5H, m), 1,58-1,49 (1 H, m), 1,42-1,3 (1 H, m), 1,26 - 1,16 (1 H, m), 1,08-0,8 (2H, m), 0,92 (6H, d, 6,7 Hz), 0,89 (3H, d, 6,5 Hz), 0,89 (3H, d, 6,9 Hz), 0,78 (3H, d, 6.9 Hz) ppm.
¹³C-NMR (100 MHz, CDCl₃, TMS): δ = 176.88 (C), 169,31 (C), 49,27 (CH), 46,94 (CH₂), 44,33 (CH), 43,64 (CH₂), 39,45 (CH₂), 34,57 (CH₂), 32,25 (CH), 28,81 (CH), 28,48 (CH), 23,81 (CH), 22,31 (CH₂), 21,41 (CH₃), 20,10 (2 x CH₃), 16,05 (CH₃) ppm.
MS (EI): *m*/*z* = 296 (M⁺, 50 %), 224 (50 %), 197 (45 %), 167 (50 %), 139 (50 %), 131 (100 %), 83 (80 %)

### Beispiel 2: Synthese von L- N^{α}-(Menthancarboxyl)glycin-N-ethylamid (Verbindung 3)

L-*N*^{α}-(Menthancarboxyl)glycinethylester ("WS 5") wurde mit KOH in Wasser verseift, das Rohprodukt mit Thionylchlorid zum Säurechlorid und das durch Eindampfen erhaltene Produkt mit Ethylaminhydrochlorid umgesetzt.

### Beispiel 3: Synthese von L-N^{α}-(Menthancarboxyl)-L-alanin-N-ethylamid (Verbindung 5)

Analog zu Verbindung 3 wurde ausgehend von L- *N*^{α}-(Menthancarboxyl)-L-alaninethylester Verbindung 5 erhalten.

### Anwendungsbeispiel 1: Kühlwirkung

Die Verbindungen wurden auf ihre sensorischen Eigenschaften, insbesondere ihre Kühlwirkung getestet. Dafür wurden sie jeweils in einer bestimmten Endkonzentration in einer aus Sucrose (Saccharose) und Wasser bereiteten Masse (Konditorenfondant, Lieferant Nordzucker AG, Nordstemmen) gelöst und in einer Runde von Experten bewertet. Die sensorischen Eindrücke wurden notiert und die Kühlwirkung anhand einer Skala von 1 (keine Kühlwirkung) bis 9 (extrem starke Kühlwirkung) bewertet.

Profil des von L-*N*^{α}-(Menthancarboxyl)glycin-isobutylamids (Beispiel 1) bei einer Konzentration von 0,05 Gew.-%, bezogen auf die Gesamtzubereitung: sehr schwach bitter, Kühlwirkung 5

### Anwendungsbeispiel 2: Aromamischung zur Erreichung einer Kühlwirkung

| **Inhaltsstoff** | **Anteil in Gew-%** |
|---|---|
| L-*N*^{α}-(Menthancarboxyl)glycin-N-ethylamid aus Beispiel 3 | 25 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischung der Komponenten wird eine stark kühlende, ansonsten jedoch nahezu geschmacks- und geruchslose, bei Raumtemperatur (20 °C) flüssige Aromamischung erhalten.

### Anwendungsbeispiel 3: Aromamischung zum Erreichen einer Kühlwirkung

| **Inhaltsstoff** | **Anteil in Gew-%** |
|---|---|
| L-*N*^{α}-(Menthancarboxyl)glycin-N-isobutylamid aus Beispiel 1 | 7,5 |
| L-Menthancarbonsäure-N-ethylamid (WS 3, z.B. Millenium) | 5 |
| L-Menthyllactat (Frescolat ML, Symrise) | 32,5 |
| O-L-Menthyl-O'-(2-hydroxyethyl)carbonat (Frescolat MGC, Symrise) | 5 |
| Propylenglycol | 50 |

Durch Mischung der Komponenten wird eine stark kühlende, ansonsten jedoch nahezu geschmacks- und geruchslose, bei Raumtemperatur (20 °C) flüssige Aromamischung erhalten.

### Anwendungsbeispiel 4: Aromamischung zum Erreichen einer Aroma- und Kühlwirkung

| **Inhaltsstoff** | **Anteil in Gew-%** |
|---|---|
| L-*N*^{α}-(Menthancarboxyl)glycin-N-ethylamid aus Beispiel 3 | 15 |
| Pfefferminzöl | 10 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischung der Komponenten wird eine stark kühlende, stark nach Pfefferminz riechende Aromamischung erhalten.

### Anwendungsbeispiel 5: Aromamischung zum Erreichen einer Kühlwirkung mit gleichzeitigem Tingling-Effekt

| **Inhaltsstoff** | **Anteil in Gew-%** |
|---|---|
| L-*N*^{α}-(Menthancarboxyl)glycin-*N*-ethylamid aus Beispiel 3 | 15 |
| Lösung aus 10 Gew.-% Pellitorin in Propylenglycol/Pfefferminzöl 1:1 | 10 |
| L-Menthyllactat (Frescolat ML, Symrise) | 65 |
| O-L-Menthyl-O'-(2-hydroxyethyl)carbonat (Frescolat MGC, Symrise) | 10 |

Durch Mischung der Komponenten wird eine stark kühlende Aromamischung erhalten, welche speichelanregend ist und einen Tingling-Effekt verursacht.

### Anwendungsbeispiel 6: Verwendung in Form einer Aromamischung in einer Zahnpasta

| | | |
|---|---|---|
| **Teil** | **Inhaltsstoff** | **Einsatz in Gew.-%** |
| A | demineralisiertes Wasser | 22,00 |
| | Sorbitol (70%) | 45,00 |
| | Solbrol^{®} M, Natriumsalz (Bayer AG, p- Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aromamischung aus Anwendungsbeispiel 2 | 1 |

Die Inhaltsstoffe der Teile A und B wurden jeweils für sich vorgemischt und zusammen unter Vakuum bei 25 - 30°C jeweils 30 min lang gut verrührt. Teil C wurde vorgemischt und zu A und B gegeben; D wurde hinzugefügt und die Mischung unter Vakuum bei 25 - 30°C weitere 30 min gut verrührt. Nach Entspannung war die Zahnpasta fertig und konnte abgefüllt werden. Bei der Verwendung der so hergestellten Zahnpasta konnte eine deutliche Kühlwirkung festgestellt werden.

### Anwendungsbeispiel 7: Verwendung als Kühlwirkstoff in einem zuckerfreien Kaugummi

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Spearmint-Pfefferminz-Eucalyptus-Aroma, enthaltend 5 Gew.-% L-*N*^{α}-(Menthancarboxyl)glycin-N-isobutylamid aus Beispiel 1 | 1 |

Teile A bis D wurden gemischt und intensiv geknetet. Die Rohmasse wurde z.B. in Form von dünnen Streifen zu verzehrsfertigen Kaugummis verarbeitet. Bei der Verwendung des so hergestellten Kaugummis konnte eine deutliche Kühlwirkung festgestellt werden.

### Anwendungsbeispiel 8: Verwendung als Kühlwirkstoff in einem Mundwasser

| **Teil** | **Inhaltsstoff** | **Einsatz in Gew.-%** |
|---|---|---|
| A | Ethanol | 10,00 |
| | Cremophor^{®} CO 40 (BASF, Detergenz) | 1,00 |
| | Benzoesäure | 0,12 |
| | Pfefferminz-Zitronenmelisse-Aroma, enthaltend 0,4 Gew.-% Pellitorin und 10 Gew.-% L-*N*^{α}-(Menthancarboxyl)glycin-N-isobutylamid aus Beispiel 1 | 0,25 |
| B | demineralisiertes Wasser | 83,46 |
| | Sorbitol, 70% | 5,00 |
| | Natriumsaccharin 450 | 0,07 |
| | L-Blue 5000 e.c., 1% in Wasser (Farbstoff) | 0,10 |

Die Inhaltsstoffe der Teile A und B wurden jeweils für sich gemischt. Teil B wurde langsam in Teil A eingerührt, bis die Mischung homogen war.
Bei der Verwendung des so hergestellten Mundwassers konnte eine deutliche Kühlwirkung festgestellt werden.

### Anwendungsbeispiel 9: Halsbonbons mit flüssig-viskoser Kernfüllung (centre-filled hard candy)

| | I (Gew.-%) | II (Gew.-%) |
|---|---|---|
| **Mischung A (Hülle) (80% der Bonbons)** | | |
| Zucker (Saccharose) | 58,12 | 49,37 |
| Glucosesirup (Feststoffgehalt 80%) | 41,51 | 49,37 |
| Aromamischung aus Anwendungsbeispiel 5 | 0,17 | 0,25 |
| l-Menthol | 0,10 | - |
| Citronenöl | 0,10 | 0,10 |
| Citronensäure | - | 0,91 |
| Total: | 100 | 100 |

| **Mischung B (Kern) (20% der Bonbons)** | | |
|---|---|---|
| High Fructose Maissirup (Gehalt an festen Zuckern 85%, knapp 15% Wasser) | 84,38 | 84,36 |
| Glycerin | 15,0 | 15,0 |
| Lecithin | 0,02 | 0,02 |
| Zimtöl | - | 0,32 |
| Spearmintöl | 0,28 | - |
| Capsaicin | 0,05 | - |
| Vanillylalkohol-n-butylether | - | 0,10 |
| Roter Farbstoff, als 5%ige wässrige Lösung | 0,20 | 0,20 |
| Vanillin | 0,07 | - |
| Total | 100 | 100 |

In Anlehnung an die in US 6,432,441 (dort Beispiel 1) sowie die in US 5,458,894 bzw. US 5,002,791 beschriebenen Verfahren wurden Bonbons mit flüssigviskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Anwendungsbeispiel 10: Kaugummi

Die Kaugummibase K2 bestand aus folgenden Zutaten: 28,5% Terpenharz, 33,9% Polyvinylacetat (MW = 14,000), 16,25% hydriertes Pflanzenöl, 5,5% Mono- und Diglyceride, 0,5% Polyisobuten (MW 75,000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis erfolgte analog zu US 6,986,907.

| | I (Gew.-%) | II (Gew.-%) | III (Gew.-%) |
|---|---|---|---|
| Kaugummibase K2 | 25,30 | 27,30 | 26,30 |
| Sorbitol | 61,48 | 59,48 | 61,80 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet | 0,50 | - | - |
| Kirscharoma, sprühgetrocknet | - | 1,20 | - |
| Aromamischung aus Anwendungsbeispiel 4, sprühgetrocknet | 1,50 | 1,80 | - |
| Aromamischung aus Anwendungsbeispiel 3 | 1,00 | - | 1,68 |

Die Kaugummis der Rezeptur (I) und (II) wurden als Streifen, die der Rezeptur (III) als Pellets ausgeformt.
Bei der Verwendung des so hergestellten Kaugummis konnte eine deutliche Kühlwirkung festgestellt werden.

### Anwendungsbeispiel 11: Gelatinekapsel zum Direktverzehr

| | I (Gew.-%) | II (Gew.-%) | III (Gew.-%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |
| Kernzusammensetzung: | | | |
| Pflanzenöl-Triglycerid (Kokosöl - Fraktion) | 79,39 | 68,40 | 58,25 |
| Zimt-Anis-Aroma | 10,00 | 20,90 | - |
| Eucalyptus-Aroma | - | - | 29,95 |
| Neotam und Aspartam | 0,01 | 0,05 | - |
| Sucralose | 0,22 | 0,30 | 0,70 |
| Aromamischung aus Anwendungsbeispiel 5 | 0,33 | - | - |
| Aromamischung aus Anwendungsbeispiel 3 | - | 0,20 | 0,60 |
| L-*N*^{α}-(Menthancarboxyl)glycin-N-ethylamid aus Beispiel 3 | - | 0,05 | - |
| (-)-Menthonglycerinacetal (Frescolat MGA) | - | 0,10 | 0,40 |
| Vanillin | 0,05 | - | 0,10 |

Die zum Direktverzehr geeigneten Gelatinekapseln I, II, III wurden gemäß WO 2004/050069 hergestellt und hatten jeweils einen Durchmesser von 5 mm, das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.
Bei der Verwendung der so hergestellten Gelatinekapseln konnte eine deutliche Kühlwirkung festgestellt werden.

### Anwendungsbeispiel 12: Kaubonbon

| | | Gew-% |
|---|---|---|
| Wasser | | 7,80 % |
| Zucker | Raffinade C4 | 42,10 % |
| Glucose Sirup | Dextrose 40 | 37,30 % |
| gehärtetes Pflanzenfett | Schmelzpunkt 32-36°C | 6,60 % |
| Lecithin | Emulgator (Sojalecithin) | 0,30 % |
| Gelatine | Schweinegelatine | 0,80 % |
| Fondant | Typ - S30 | 4,80 % |
| Himbeeraroma | | 0,22 % |
| Aromamischung aus Anwendungsbeispiel 3 | | 0,08 % |

Herstellungshinweise:
a) Gelatine mit Wasser (1,8 fache Menge der Gelatine) bei 70°C 2 Stunden quellen lassen;
b) Zucker, Sirup, Wasser, Fett und Lecithin auf 123°C kochen;
c) Gelatinelösung langsam mit dem Kochansatz vermischen;
d) Aroma aus Beispiel 2 und optional Farbe unterrühren;
e) resultierende Masse auf einem Kühltisch auf ca. 70°C temperieren, danach Fondant zugeben und auf einer Ziehmaschine ca. 3 Minuten belüften;
f) Kaubonbonmasse anschließend schneiden und verpacken.
Beim Verzehr der Kaubonbons wird während des Kauens ein frischer, kühlender Himbeergeschmack wahrgenommen.

### Anwendungsbeispiel 13: Extrudat

| | | |
|---|---|---|
| Glukosesirup, sprühgetrocknet (DE-Wert: 31-34) | Glucidex IT33W (Firma Roquette) | 62,0 % |
| Maltodextrin (DE-Wert: 17-20) | (Firma Cerestar) | 28,4% |
| Emulgator Monomuls | Emulgator auf der Basis von gehärtetem Palmöl; Schmelzpunkt: 64°C, (Firma Grünau) | 1,8 % |
| Dextrosemonohydrat (DE-Wert: 99,5) | Dextrose, kristallwasserhaltig (Firma Cerestar) | 1,8 % |
| Wasser | | 2,0 % |
| Orangen-Vanillearoma | | 3,2 % |
| Aromamischung aus Anwendungsbeispiel 4 | | 0,8 % |

Herstellungshinweis (siehe auch WO 03/092412):

Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

### Anwendungsbeispiel 14: Wirbelschichtgranulate

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aromamischung aus Anwendungsbeispiel 4, 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Anwendungsbeispiel 15: Teebeutel mit Rooibos bzw. schwarzem Tee und Extrudaten aus Auswendungsbeispiel 13 bzw. Granulaten aus Anwendungsbeispiel 14

Jeweils 800 g Rotbuschtee (Rooibos-Tee) wurden einmal mit 33 g der Extrudate aus Anwendungsbeispiel 13 und einmal mit 30 g Granulaten aus Anwendungsbeispiel 14 gemischt, portioniert und anschließend in Teebeutel abgefüllt.

Jeweils 800 g schwarzer Tee (Blattgrad Fannings) wurden einmal mit 33 g der Extrudate aus Anwendungsbeispiel 13 und einmal mit 30 g Granulaten aus Anwendungsbeispiel 14 gemischt, portioniert und anschließend in Teebeutel abgefüllt.

Die in den Anwendungsbeispiel gefundenen Effekte lassen sich sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen, Gemische und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen, Mischungen und Gemische aufgefasst werden muss. Auch die Konzentration der verwendeten erfindungsgemäßen Verbindung, Mischung oder des verwendeten Gemisches ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile sind in der oben stehenden Beschreibung offenbart.

## Patentansprüche

1. Verbindung oder Gemisch von Verbindungen der Formel (I): 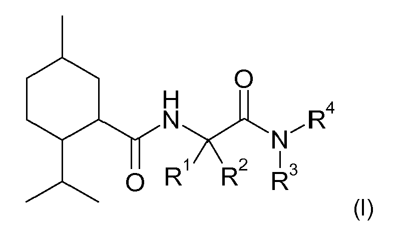 wobei
R¹ und R² jeweils unabhängig voneinander Wasserstoff oder einen
(a) linearen, verzweigten oder cyclischen,
(b) gesättigten oder ungesättigten,
(c) unsubstituierten, ein- oder mehrfach substituierten Kohlenwasserstoffrest mit
(d) 1 bis 5 Kohlenstoffatomen bedeuten, wobei
(e) die Kohlenwasserstoffreste R¹ und R² auch über eine Einfachbindung oder über eine Gruppe -O-, -S- oder -NH- verknüpft sein können und dabei bevorzugt einen 3- bis 7-gliedrigen Ring ausbilden,
und
R³ und R⁴ jeweils unabhängig voneinander Wasserstoff oder einen
(a) linearen, verzweigten oder cyclischen,
(b) gesättigten oder ungesättigten Kohlenwasserstoffrest mit
(c) 1 bis 10 Kohlenstoffatomen bedeuten, wobei
(d) die Kohlenwasserstoffreste R³ und R⁴ auch über eine Einfachbindung oder über eine Gruppe -O-, -S- oder -NH- verknüpft sein können und dabei bevorzugt einen 3 bis 7-gliedrigen heterocyclischen Ring ausbilden,
oder
R³ und R⁴ unabhängig voneinander jeweils Wasserstoff oder einen
(a) linearen oder verzweigten,
(b) durch eine Gruppe R⁵-X- substituierten Kohlenwasserstoffrest mit
(c) 1 bis 10 Kohlenstoffatomen bedeuten, wobei jedes X falls vorhanden jeweils unabhängig von dem anderen X falls vorhanden
(d) Sauerstoff, Schwefel oder eine Gruppe -NR⁶- darstellt,
und
wobei jeder R⁵ und jeder R⁶ falls vorhanden jeweils unabhängig voneinander Wasserstoff oder einen
(a) linearen, verzweigten oder cyclischen,
(b) gesättigten oder ungesättigten Kohlenwasserstoffrest mit
(c) 1 bis 5 Kohlenstoffatomen bedeuten,
und
wobei bevorzugt die jeweilige Verbindung der Formel (I) einzeln oder eine der Verbindungen der Formel (I) im Gemisch von Verbindungen der Formel (I) unabhängig von den anderen Verbindungen der Formel (I) als einzelnes Stereoisomer (Enantiomere und Diastereomere) oder als bestimmtes Gemisch verschiedener Stereoisomere vorliegt.

2. Verbindung oder Gemisch von Verbindungen nach Anspruch 1, wobei in der Formel (I)
R¹ Wasserstoff bedeutet
und/oder
R² Wasserstoff oder einen
(a) linearen, verzweigten oder cyclischen,
(b) gesättigten oder ungesättigten,
(c) unsubstituierten, ein- oder mehrfach substituierten Kohlenwasserstoffrest mit
(d) 1 bis 5 Kohlenstoffatomen bedeutet
und/oder
R³ und R⁴ jeweils unabhängig voneinander Wasserstoff oder einen
(a) linearen, verzweigten oder cyclischen Kohlenwasserstoffrest mit
(b) 1 bis 10 Kohlenstoffatomen bedeuten, wobei
(c) bevorzugt die Kohlenwasserstoffreste R³ und R⁴ über eine Einfachbindung oder über eine Gruppe -O-, oder -NH- verknüpft sind und dabei einen Azirin-, Azetidin-, Pyrrolidin-, Imidazolidin, Piperidin-, Pyrazolidin-, Diazan- oder Morpholin-Ring ausbilden
oder
R³ und R⁴ unabhängig voneinander jeweils Wasserstoff oder einen
(a) linearen oder verzweigten,
(b) durch eine Gruppe R⁵-X- substituierten Kohlenwasserstoffrest mit
(c) 1 bis 10 Kohlenstoffatomen bedeuten, wobei bevorzugt jedes X falls vorhanden jeweils unabhängig von dem anderen X falls vorhanden
(d) Sauerstoff, Schwefel oder eine Gruppe -NR⁶- darstellt und wobei bevorzugt jeder R⁶ falls vorhanden Wasserstoff bedeutet.

3. Verbindung oder Gemisch von Verbindungen nach Anspruch 1 oder 2, wobei die Verbindung der Formel (I) oder eine Verbindung der Formel (I) im Gemisch von Verbindungen der Formel (I) unabhängig von den anderen Verbindungen der Formel (I) hinsichtlich ihrer Stereochemie entsprechend der Formel (Ia) 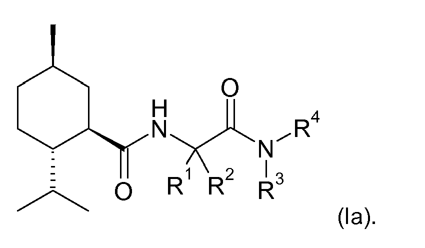 zu 90 -100 Mol-% festgelegt ist, wobei
in der Formel (la) die Reste R¹, R², R³ und R⁴ jeweils die Bedeutung der entsprechenden Reste der Formel (I) haben
und wobei bevorzugt alle Verbindungen der Formel (I) in dem Gemisch hinsichtlich ihrer Stereochemie zu 90 -100 Mol-% entsprechend der Formel (la) festgelegt sind.

4. Verbindung oder Gemisch von Verbindungen nach einem der vorangehenden Ansprüche, wobei in den Formeln (I) und (la)
R¹ Wasserstoff bedeutet
und/oder
R² Wasserstoff oder einen Methyl-, Ethyl-, Propyl-, 2-Propyl-, 2-Methylpropyl-, 2-Butyl-, 2-Methylbutyl-, 4-Aminobutyl-, 3-Guanidinopropyl, 3-Aminopropyl-, 3-Ureidopropyl-, Indol-3-ylmethyl-, 2-Carboxyethyl-,
Carboxymethyl-, 2- (Aminocarbonyl)ethyl-, (Aminocarbonyl)methyl-, Thiomethyl-, 2-Thioethyl-, 2-Methylthioethyl-, Hydroxymethyl-, 1-Hydroxyethyl-, Phenylmethyl- oder 4-Hydroxyphenylmethyl-Rest bedeutet
und/oder
R³ Wasserstoff oder einen Methyl- oder Ethyl- Rest bedeutet,
und/oder
R⁴ Wasserstoff oder einen Methyl-, Ethyl-, 2-Propyl-, Cyclopropyl-, Propyl-, Butyl-, Cyclobutyl-, 2-Butyl-, 2-Methylprop-1-yl, 2-Methylprop-2-yl-, Pentyl-, Cyclopentyl-, 2-Pentyl-, 3-Pentyl-, Hexyl-, Cyclohexyl-, 2-Hexyl- und 3-Hexyl-, Heptyl-, Octyl-, Isooctyl-, Nonyl- oder Decyl-Rest bedeutet,
oder
R⁴ einen durch eine Gruppe R⁵-X- substituierte n Methylen -, Ethylen-, 1,2-Propylen-, 1,3-Propylen-, 1,2-Butylen-, 1,3-Butylen-, 1,4-Butylen-, 1,2-Pentylen-, 1,3-Pentylen-, 1,4-Pentylen- oder 1,5-Pentylen-Rest bedeutet, wobei X Sauerstoff darstellt und R⁵ Wasserstoff oder einen Methyl- oder Ethyl-Rest bedeutet,
und/oder
wobei bevorzugt die Verbindung oder die Verbindungen der Formel (I) zu 90 - 100 Mol-% hinsichtlich ihrer Stereochemie entsprechend der Formel (la) festgelegt sind.

5. Verbindung oder Gemisch von Verbindungen nach einem der vorangehenden Ansprüche, wobei in den Formeln (I) und (la)
R² Wasserstoff oder einen Methyl-, Ethyl-, Propyl- oder 2-Propyl -Rest bedeutet und/oder
R⁴ Wasserstoff oder einen Methyl-, Ethyl-, 2-Propyl-, Cyclopropyl-, Propyl-, Butyl-, Cyclobutyl-, 2-Butyl-, 2-Methylprop-1-yl, 2-Methylprop-2-yl-, Pentyl-, Cyclopentyl-, 2-Pentyl-, 3-Pentyl-, Hexyl-, Cyclohexyl-, 2-Hexyl- und 3-Hexyl-, Heptyl-, Octyl-, Isooctyl-, Nonyl- oder Decyl-Rest bedeutet
oder
R⁴ einen durch eine Gruppe R⁵-X- substituierte n Methylen -, Ethylen-, 1,2-Propylen- oder 1,3-Propylen-Rest bedeutet,
wobei X Sauerstoff darstellt und
R⁵ Wasserstoff oder einen Methylrest bedeutet.

6. Verbindung oder Mischung nach einem der vorangehenden Ansprüche , bestehend aus einer Verbindung der Formel (I) oder unfassend Verbindungen der Formel (I) ausgewählt aus der Gruppe bestehend aus
L- *N*^{α}-(Menthancarboxyl)glycin-*N*-isobutylamid,
L- *N*^{α}-(Menthancarboxyl)glycin-*N*,*N*-dimethylamid,
L- *N*^{α}-(Menthancarboxyl)glycin-*N*-ethylamid,
L- *N*^{α}-(Menthancarboxyl)glycin-*N*-ethanolamid und
L- *N*^{α}-(Menthancarboxyl)-L-alanin-*N*-ethylamid.

7. Mischung bestehend aus/umfassend
(a) einer/eine Verbindung oder einem/ein Gemisch von Verbindungen nach einem der vorangehenden Ansprüche
sowie
(b) einem/einen oder mehreren/mehrere weitere Stoffe mit physiologischer Kühlwirkung,
wobei der weitere Stoff bzw. einer, mehrere oder sämtliche der weiteren Stoffe (i) einen geschmacklichen Effekt verursachen oder (ii) keinen geschmacklichen Effekt verursachen,
und/oder
(c) einem/einen oder mehreren/mehrere Aromastoffen/Aromastoffe ohne physiologische Kühlwirkung
und/oder
(d) einem/einen oder mehreren/mehrere trigeminal oder mundwässernd wirksame(n) Stoffe(n) ohne physiologische Kühlwirkung.

8. Mischung nach Anspruch 7, umfassend
im Bestandteil (b) einen oder mehrere weitere Stoffe mit physiologischer Kühlwirkung ohne geschmacklichen Effekt
und/oder
im Bestandteil (c) einen oder mehrere Aromastoffe ohne physiologische Kühlwirkung
und/oder
eine oder mehrere Verbindungen, der oder die unabhängig von einander oder gemeinsam zusätzlich einen geschmacksmodulierenden Effekt und/oder einen trigeminalen und/oder einen mundwässernden Reiz verursachen.

9. Der Ernährung, der Mundhygiene oder dem Genuss dienende Zubereitung oder pharmazeutische oder kosmetische Zubereitung, umfassend eine zum Erreichen einer physiologischen Kühlwirkung auf der Haut und/oder Schleimhaut ausreichende Menge
(a) einer Verbindung oder eines Gemisches nach einem der Ansprüche 1 bis 6 oder
(b) eine Mischung nach einem der Ansprüche 7 oder 8.

10. Verwendung
(a) einer Verbindung oder eines Gemisches nach einem der Ansprüche 1 bis 6 oder
(b) einer Mischung nach einem der Ansprüche 7 oder 8 oder
(c) einer Zubereitung nach Anspruch 9
zum Erzeugen einer Kühlwirkung auf der Haut oder einer Schleimhaut zu
(i) anderen als therapeutischen Zwecken oder
(ii) zum Herstellen eines Arzneimittels.

11. Verwendung nach Anspruch 10 zum Herstellen eines Arzneimittels, wobei das herzustellende Arzneimittel der Bekämpfung oder Linderung von Husten-, Schnupfen-, Entzündungs-, Halsschmerz- oder Heiserkeitssymptomen dient.

12. Therapeutisches oder nicht-therapeutisches Verfahren zum Erreichen einer physiologischen Kühlwirkung auf der Haut und/oder einer Schleimhaut, mit folgendem Schritt:
- Applizieren einer zum Erreichen einer physiologischen Kühlwirkung ausreichenden Menge
(a) einer Verbindung oder eines Gemisches nach einem der vorangehenden Ansprüche 1 bis 6 oder
(b) einer Mischung nach einem der Ansprüche 7 oder 8 oder
(c) einer Zubereitung nach Anspruch 9
auf die Haut und/oder eine Schleimhaut.

13. Verfahren zur Herstellung einer Verbindung nach einem der Ansprüche 1 bis 6 umfassend die Schritte:
(a) Bereitstellen einer entsprechenden *N*^{α}-(Menthancarbonyl)aminosäure, eines entsprechenden *N*^{α}-(Menthancarbonyl)aminosäurechlorids oder eines anderen entsprechenden aktivierten *N*^{α}-(Menthancarbonyl)aminosäurederivats,
(b) Bereitstellen eines entsprechenden Amins oder eines entsprechenden Salzes und
(c) Umsetzen der bereitgestellten Verbindungen miteinander, so dass eine Verbindung nach einem der Ansprüche 1 bis 6 entsteht.
